# EUROPEAN PATENT APPLICATION

(11) **EP 1 555 264 A1**
(43) Date of publication of application: **20.07.2005**
(21) Application number: 04000762.7
(22) Date of filing: 15.01.2004
(51) Int. Cl.: C07D 417/12, C07D 417/04

(54) **Five-membered heterocyclic compounds as inhibitors of SRC family protein kinase.**

(71) Applicant: Sireen AG, 82152 Martinsried (DE)
(72) Inventor: Miksch, Christin, 81375 München (DE); Bosse, Folkert, 81241 München (DE); Hohlfeld, Thomas, 81475 München (DE); Scheufler, Clmenens, 79576 Weil am Rhein (DE); Obermeier, Axel, 81379 München (DE); Moarefi, Ismail Dr., 80337 München (DE); Kostka, Thomas, 86919 Utting (DE); Monse, Barbara, 85258 Weichs (DE)
(74) Representative: Jehle, Volker Armin

(57) **Abstract**

The present invention refers to novel substituted aromatic heteroaryl derivatives of formula (I). 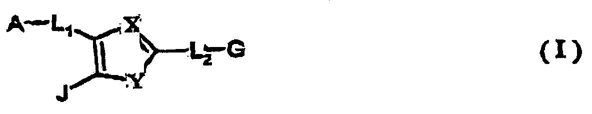 with the definitions of A, L₁, L₂, G, J, X and Y according to claim 1.
These novel compounds are useful for the inhibition of protein kinases, particularly of the inhibition of Src family protein kinases. Methods for inhibiting kinases by contacting kinases with these novel compounds are disclosed. In another embodiment the present invention refers to pharmaceutical compositions containing these novel compounds and their use for the preparation of medicaments for treating diseases or disorders associated with unphysiological activity of kinases in the body, particularly for the treatment of cancer, immunosuppression, and osteoporosis.

## Description

The present invention refers to novel compounds based on a substituted aromatic heteroaryl ring system. These novel compounds are useful for the inhibition of protein kinases, particularly of the inhibition of Src family protein kinases. Methods for inhibiting kinases by contacting kinases with these novel compounds are disclosed. In another embodiment the present invention refers to pharmaceutical compositions containing these novel compounds and their use for the preparation of medicaments for treating diseases or disorders associated with unphysiological activity of kinases in the body, particularly for the treatment of cancer, immunosuppression, and osteoporosis.

Cellular protein targets, which play a key role for cellular processes, are the members of the kinase family. These kinases are implicated in e.g. cancer, immune system dysfunction and bone remodelling diseases. For general reviews, see Thomas and Brugge, Annu. Rev. Cell Dev. Biol. (1997) 13, 513; Lawrence and Niu, Pharmacol. Ther. (1998) 77, 81; Tatosyan and Mizenina, Biochemistry (Moscow) (2000) 65, 49; Boschelli et al., Drugs of the Future 2000, 25(7), 717, (2000). Kinases phosphorylate target proteins at particular sites, primarily at tyrosine, threonine or serine residues. By their phosphorylation activity kinases may transduce signals and may be part of a signalling cascade resulting finally in a cellular response to external stimuli.

As an example, the Src protein family belongs to the kinase protein family. The Src family consists of the following nine kinases in mammals: Src, Fyn, Yes, Fgr, Lyn, Hck, Lck, Blk and Yrk. These are nonreceptor protein kinases that range in molecular mass from 52 to 62 kD. All are characterized by a common structural organization that is comprised of six distinct functional domains: Src homology domain 4 (SH4), a unique domain, SH3 domain, SH2 domain, a catalytic domain (SH1), and a C-terminal regulatory region (Tatosyan et al. Biochemistry (Moscow) 65, 49-58 (2000)). Src family kinases are for example involved in cell division (probably through interaction with different growth factor receptors), as well as cell shape changes, adhesion and motility through regulation of integrin signalling and cytoskeletal architecture (Roche et al., 1995, Science 269, 1567-9; Mao et al., 1997, Oncogene 15, 3083-90; Parsons and Parsons, 1997, Curr. Opin. Cell Biol. 9, 187-92; Abu-Ghazaleh et al., 2001, Biochem. J. 360, 255-64; Belsches-Jablonski et al., 2001, Oncogene 20, 1465-75; Avizienyte et al., 2002, Nat. Cell Biol. 4, 632-638; Frame, 2002, BBA 1602, 114-30; Kitagawa et al., 2002, J. Biol. Chem. 277, 366-71).

Because of its functions, a number of studies have implicated Src in the generation and/or progression of several types of cancer, including breast, hepatic, pancreatic and ovarian cancer and in particular several kinds of leukemia and lymphomas as well as colon cancer (Cartwright et al., 1990, Proc. Natl. Acad. Sci. USA 87, 558-62; Lynch et al., 1993, Leukemia 7, 1416-22; Pories et al., 1998, Gastroenterology 114, 1287-95; Frame, 2002, BBA 1602, 114-30; Talamonti et al., J. Clin. Invest., 91, 53 (1993); Lutz et al., Biochem. Biophys. Res. 243, 503 (1998); Rosen et al., J. Biol. Chem., 261, 13754 (1986); Bolen et al., Proc. Natl. Acad. Sci. USA, 84, 2251 (1987); Masaki et al., Hepatology, 27, 1257 (1998); Biscardi et al., Adv. Cancer Res., 76, 61 (1999); Lynch et al., Leukemia, 7, 1416 (1993)) and other disorders.

An unambiguous correlation between Src kinase activity and advancing tumor stage in colon cancer has been demonstrated (Talamonti et al., J. Clin. Invest., 91, 53 (1993)). It was shown that there is a strong positive correlation between Src activity and tumor progression from non-malignant polyps towards invasive cancers and metastatic foci. Therefore, activation of Src kinase in primary colorectal carcinoma entails a poor clinical prognosis (Aligayer et al., 2002, Cancer 94, 344-51). A reduced activation of Src in human colon and ovarian cancer cells was shown to reduce their tumorigenicity, suggesting that Src inhibitors could have a potential as anti-cancer drugs (Wiener et al., Clin. Cancer Res., 5, 2164 (1999); Staley et al., Cell Growth Diff., 8, 269 (1997)). Colon cancer is a common disease which leads to death in ~50% of the cases as a consequence of metastasis. Thus, therapeutic substances are highly desired for more successful treatment strategies of a variety of diseases or disorders, in particular cancer. As a consequence, there is a long lasting need in the art for kinase inhibitors that allow to identify and characterise compounds modulating the activity of kinases.

Although a variety of compounds have been identified, which inhibit certain kinases, e.g. tyrosine kinase inhibitors, it is of particular interest to identify and characterize novel compounds with advantageous properties (e.g. higher hydrophilicity, cell permeability, less side effects, bioavailability, to be administered orally, selectivity, therapeutical alternative, alternative pharmacological profile and/or other advantages etc.).

It is the object of the present invention to provide novel compounds which do not exhibit certain deficiencies of the prior art compounds. It is a further object of the invention to provide these compounds according to the invention for therapeutical use for the treatment of a variety of kinase associated diseases.

According to claim 1 the compounds of the invention have general formula (I). 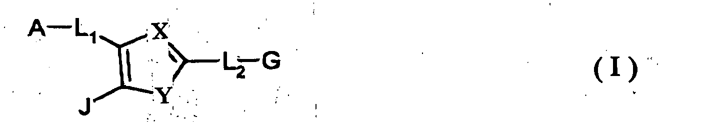 In formula (I),
X represents N, O, C or S
Y represents S, NH, C or O
L¹ represents
a chemical bond;
carbonyl;
-(CH₂)ₐ- wherein
a is 1, 2, 3, 4 or 5;
-CH₂O-;
-OCH₂-;
-O- or -S-;
-N(R¹)- wherein
R¹ represents H, C₁₋₄ alkyl or NH;
-NHC(O)-; 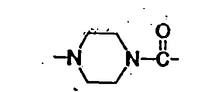 -CH₂NHC(O)-;
L² represents
a chemical bond;
-(CH₂)ₐ-;
-CH₂O-;
-N(R¹)-;
-NH(CH₂)ₐ-;
-N(CO)R¹-; or
-NHC(O)NH-;
J represents
H;
C₁₋₄ alkyl, wherein C₁₋₄ may be substituted by at least one halogen; or
halogen; and
A represents a (hetero)cyclic, aromatic or non-aromatic optionally substituted ring system;
G represents a (hetero)cyclic, aromatic or non-aromatic optionally substituted ring system or
L¹, A and J form together a cyclic or heterocyclic mono-, bi- or tricyclic ring system
or a pharmaceutically acceptable salt thereof.

It is preferred to combine L¹, A and J, whereby a cyclic or heterocyclic mono-, bi- or tricyclic ring system is formed by L¹, A and J. This ring system may be an aromaric or a nonaromatic ring system fused with the central heteroaryl C₅ ring of formula (I). E.g. the fused ring systems may form a C₉ - C₁₇ heteroaryl ring, in particular a C₉, C₁₃ or C₁₇ heteroaryl ring, e.g. benzothiazole, benzooxazole, chinoxaline, indole, chinoline, purin, isochinoline.

For convenience, certain terms employed in the specification, examples, and appended claims are collected here. The term "agonist" of an enzyme refers to a compound that binds to the enzyme and stimulates the action of the naturally occurring enzyme, or a compound which mimics the activity of the naturally occurring enzyme. The term "antagonist" of an enzyme refers to a compound that binds to the enzyme and inhibits the action of the naturally occurring enzyme. The term "analog" of a compound refers to a compound having a some structural similarity to a particular compound and having essentially the same type of biological activity as the compound.

The term "derivative" of a compound refers to another compound which can be derived, e.g., by chemical synthesis; from the original compound. Thus a derivative of a compound has certain structural similarities with the original compound.

"Disease associated with an abnormal activity or level of a kinase" refers to diseases in which an abnormal activity or protein level of a kinase is present in certain cells, and in which the abnormal activity or protein level of the kinase is at least partly responsible for the disease.

A "disease associated with a kinase" refers to a disease that can be treated with a kinase inhibitor, such as the compounds disclosed herein.

A "kinase" refers to an enzyme having a protein kinase activity.

"Kinase activity" refers to the activity of an enzyme to phosphorylate a substrate, particularly a protein, whereby a phosphate is covalently coupled to one or more substrate amino acids, particularly threonine, tyrosine or serine.

A "kinase inhibitor" is a compound which inhibits at least part of the activity of a kinase in a cell. The inhibition can be at least about 20%, preferably at least about 40%, even more preferably at least about 50/o, 70%, 80%, 90%, 95%, and most preferably at least about 98% of the activity of the kinase.

A "patient" or "subject" to be treated by the subject method can mean either a human or non-human animal.

"Treating" a disease refers to preventing, curing or improving at least one symptom of a disease.

The following definitions pertain to the chemical structure of compounds:

The term "heteroatom" as used herein means an atom of nitrogen, oxygen, or sulfur.

The term "alkyl" refers to the radicals of saturated aliphatic groups, including straight-chain alkyl groups and branched-chain alkyl groups.

The term "cycloalkyl" (alicyclic) refers to radicals of cycloalkyl compounds, examples being cyclopropyl, cyclobutyl, cyclopentyl, cyclohexyl, etc.

The term "aralkyl", as used herein, refers to an alkyl group substituted with an aryl group (e.g., an aromatic or heteroaromatic group).

The terms "alkenyl" and "alkynyl" refer to unsaturated aliphatic groups that contain at least one double or triple bond respectively.

Unless the number of carbons is otherwise specified, "lower alkyl" as used herein means an alkyl group but having from one to six carbons, preferably from one to four carbon atoms in its backbone structure. Likewise, "lower alkenyl" and "lower alkynyl" have similar chain lengths. Preferred alkyl groups are lower alkyls.

The term "aryl" as used herein means an aromatic group of 6 to 14 carbon atoms in the ring(s), for example, phenyl and naphthyl. As indicated, the term "aryl" includes polycyclic ring systems having two or more rings in which two or more carbons are common to two adjoining rings (the rings are "fused rings") wherein at least one of the rings is aromatic.

The term "heteroaryl" as used herein means an aromatic group which contains at least one heteroatom in at least one ring. Typical examples include 5-, 6- and 7-membered single-ring aromatic groups that may include from one to four heteroatoms. Examples include pyrrole, furan, thiophene, imidazole, oxazole, thiazole, triazole, tetrazole, pyrazole, pyridine, pyrazine, pyridazine and pyrimidine, and the like. These aryl groups may also be referred to as "aryl heterocycles" or "heteroaromatics."

The terms ortho, meta and para apply to 1,2-, 1,3- and 1,4-disubstituted benzenes or other ring systems, respectively. For example, the names 1,2-dimethylbenzene and orthodimethylbenzene are synonymous.

The terms "alkoxyl" or "alkoxy" as used herein refer to moiety in which an alkyl group is bonded to an oxygen atom, which is in turn bonded to the rest of the molecule. Examples are methoxy, ethoxy, propyloxy, tert-butoxy, etc.

As used herein, the term "nitro" means -NO₂; the term "halogen" designates -F, -Cl, - Br or -I; the term "sulfhydryl" means -SH; the term "hydroxyl" means -OH; and the term "sulfonyl" means -SO₂-.

The terms triflyl, tosyl, mesyl, and nonaflyl are art-recognized and refer to trifluoromethanesulfonyl, p-toluenesulfonyl, methanesulfonyl, and nonafluorobutanesulfonyl groups, respectively. The terms triflate, tosylate, mesylate, and nonaflate are art-recognized and refer to trifluoromethanesulfonate ester, p-toluenesulfanate ester, methanesulfonate ester, and nona-fluorobutanesulfonate ester functional groups and molecules that contain said groups, respectively.

The abbreviations Me, Et, Ph, Tf, Nf, Ts, Ms represent methyl, ethyl, phenyl, trifluoromethanesulfonyl, nonafluorobutanesulfonyl, p-toluenesulfonyl and methanesulfonyl, respectively. A more comprehensive list of the abbreviations utilized by organic chemists of ordinary skill in the art appears in the first issue of each volume of the Journal of Organic Chemistry;(i.e., J. Org. Chem. 2002, 67(1), 24A. The abbreviations contained in said list, and all abbreviations utilized by organic chemists of ordinary skill in the art are hereby incorporated by reference.

As used herein, the definition of each expression, e.g. alkyl, m, n, etc., when it occurs more than once in any structure, is intended to be independent of its definition elsewhere in the same structure.

It will be understood that "substitution" or "substituted with" includes the implicit proviso that such substitution is in accordance with permitted valence of the substituted atom and the substituent, and that the substitution results in a stable compound, e.g., which does not spontaneously undergo transformation such as by rearrangement, cyclization, elimination, etc.

As used herein, the term "substituted" is contemplated to include all permissible substituents of organic compounds. In abroad aspect, the permissible substituents include acyclic and cyclic, branched and unbranched, carbocyclic and heterocyclic, aromatic and nonaromatic substituents of organic compounds. Illustrative substituents include, for example, those described herein above. The permissible substituents can be one or more and the same or different for appropriate organic compounds. For purposes of this invention, the heteroatoms such as nitrogen may have hydrogen substituents and/or any permissible substituents of organic compounds described herein which satisfy the valences of the heteroatoms.

The phrase "protecting group" as used herein means temporary substituents which protect a potentially reactive functional group from undesired chemical transformations. Examples of such protecting groups include esters of carboxylic acids, silyl ethers of alcohols, and acetals and ketals of aldehydes and ketones, respectively. The field of protecting group chemistry has been reviewed (Greene, T.W.; Wuts, P,G.M. Protective Groups in Organic Synthesis, 3'° ed.; Wiley: New York, 1999).

Certain compounds of the present invention may exist in particular geometric or stereoisomeric forms. The present invention contemplates all such compounds, including cis- and trans-isomers, R- and S-enantiomers, diastereomers, (D)-isomers, (L)-isomers, the racemic mixtures thereof, and other mixtures thereof, as falling within the scope of the invention. Additional asymmetric carbon atoms may be present in a substituent such as an alkyl group. All such isomers, as well as mixtures thereof, are intended to be included in this invention.

If, for instance, a particular enantiomer of a compound of the present invention is desired, it may be prepared by asymmetric synthesis, oz by derivatization with a chiral auxiliary, where the resulting diastereomeric mixture is separated and the auxiliary group cleaved to provide the pure desired enantiomers. Alternatively, where the molecule contains a basic functional group, such as amino, or an acidic functional group, such as carboxyl, diastereomeric salts are formed with an appropriate optically-active acid or base, followed by resolution of the diastereomers thus formed by fractional crystallization or chromatographic means well known in the art, and subsequent recovery of the pure enantiomers. Compounds may contain a basic functional group, such as amino or alkylamino, and are, thus, capable of forming pharmaceutically acceptable salts with pharmaceutically acceptable acids.

The term "pharmaceutically acceptable salts" in this respect, refers to the relatively nontoxic, inorganic and organic acid addition salts of compounds of the present invention. These salts can be prepared in situ during the final isolation and purification of the compounds of the invention, or by separately reacting a purified compound of the invention in its free base form with a suitable organic or inorganic acid, and isolating the salt thus formed. Representative salts include the hydrobromide, hydrochloride, trifluoroacetic acid, propionic acid and butyric acid, aliphatic hydroxy acids such as lactic acid, citric acid, tartaric acid or malic acid, dicarboxylic acids such as maleic acid or succinic acid, aromatic carboxylic acids such as benzoic acid, p-chlorobenzoic acid, diphenylacetic acid or triphenylacetic acid, aromatic hydroxy acids such as o-hydroxybenzoic acid, p-hydroxybenzoic acid, 1-hydroxynaphthalene-2-carboxylic acid or 3-hydroxynaphthalene-2-carboxylic acid, and sulfonic acids such as methanesulfonic acid or benzenesulfonic acid. Furthermore, pharmaceutically acceptable salts are sulfate, bisulfate, phosphate, nitrate, acetate, valerate, oleate, palmitate, stearate, laurate, benzoate, lactate, phosphate, tosylate, citrate, maleate, fumarate, succinate, tartrate, napthylate, mesylate, glucoheptonate, lactobionate, and laurylsulphonate salts and the like. (See, for example, Berge et al. (1977) "Pharmaceutical Salts", J. Pharm. Sci. 66:1-19). Pharmaceutically acceptable salts of the subject compounds include the conventional nontoxic salts or quaternary ammonium salts of the compounds, e.g., from non-toxic organic or inorganic acids. For example, such conventional nontoxic salts include those derived from inorganic acids, such as hydrochloric, hydrobromic, sulfuric, sulfamic, phosphoric, nitric, and the like; and the salts prepared from organic acids such as formic, acetic, propionic, succinic, glycolic, stearic, lactic, malic, tartaric, citric, ascorbic, palmitic, maleic, hydroxymaleic, phenylacetic, glutamic, benzoic, salicyclic, sulfanilic, 2-acetoxybenzoic, fumaric, toluenesulfonic, methanesulfonic, ethane disulfonic, oxalic, isothionic, and the like. These salts may be prepared from compounds of formula I by known salt-forming procedures.

In other cases, the compounds of the present invention may contain one or more acidic functional groups and, thus, are capable of forming pharmaceutically acceptable salts with pharmaceutically acceptable bases. These salts can be prepared in situ during the final isolation and purification of the compounds, or by separately reacting the purified compound in its free acid form with a suitable base, such as the hydroxide, carbonate or bicarbonate of a pharmaceutically acceptable metal cation, with ammonia, or with a pharmaceutically- acceptable organic primary, secondary or tertiary amine. Representative alkali or alkaline earth salts include the lithium, sodium, potassium, calcium, magnesium, and aluminum salts and the like, Representative organic amines useful for the formation of base addition salts include ethylamine, diethylamine, ethylenediamine, ethanolamine, diethanolamine, piperazine and the like. (see, for example, Berge et al., supra).

Contemplated equivalents of the compounds described above include compounds which otherwise correspond thereto, and which have the same general properties thereof (e.g., functioning as kinase inhibitors), wherein one or more simple variations of substituents are made which do not adversely affect the efficacy of the compound in binding to a soluble or membrane residing kinase. In general, the compounds of the present invention may be prepared by the methods illustrated in the general reaction schemes as, for example, described below, or by modifications thereof, using readily available starting materials, reagents and conventional synthesis procedures. In these reactions, it is also possible to make use of variants which are in themselves known, but are not mentioned here.

In a preferred embodiment compounds of the present invention have a structure according to formula (I), wherein A and G are independently from each other a mono-, bi- or polycyclic ring system.

In a further preferred embodiment compounds of the present invention have a structure according to formula (I) wherein A and G represent independently from each other a mono- or bicyclic or polycyclic aryl or heteroaryl ring system, e.g. C₇-C₂₄ alkaryl, C₄-C₂₃ alkheteroaryl or C₆-C₁₄ aryl.

In a further preferred embodiment compounds of the present invention have a structure according to formula (I), wherein A and G are independently from each other an aromatic ring system selected from a group consisting of: benzene, pyridine, furane, pyrrole, thiophene, pyrazole, imidazole, thiazole, oxazole, pyridazine, pyrimidine, pyrazine, naphthaline, chinoline, isochinoline, indole, purine, benzofuran, phenanthrene, benzooxazole, benzothiophene, benzopyrazole, benzoimidazole,

Particularly preferred are compounds, wherein A represents 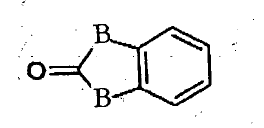 whereby B represents NH, C, S or O.

Hereby, still further preferred are compounds according to the invention, whereby A represents 3H-Benzooxazole-2-one, Benzo(1,3)dioxol-2-one, Benzol(1,3)oxathiol-2-one, 3H-Benzofuran-2-one, 1,3-Dihydro-benzoimidazole-2-one, 3H-Benzothiazole-2-one, 1,3-Dihydro-indole-2-one, 3H-Benzothiazole-2-one, 3H-Benzo(b)thiophen-2-one or Indan-2-one.

In a still further preferred embodiment compounds of the present invention have a structure according to formula (I), wherein A and G are independently from each other C₃₋₁₀ cycloalkyl, C₃₋₈ heterocycloalkyl, C₅₋₆ (hetero)cycloalkenyl, C₆₋₁₂ (hetero)bicyloalkyl or C₆₋₁₂ (hetero)bicycloalkenyl.

In a still further preferred embodiment compounds of the present invention have a structure according to formula (I), wherein A and G are independently from each other substituted by one or more substituent(s) selected from the group consisting of:
C₁₋₆ alkyl;
C₁₋₄ haloalkyl;
OR¹;
C₃₋₆ cycloalkyl;
halogen;
phenyl optionally substituted by halogen;
NO₂;
CN;
-N(R³)₂; wherein
   R³ represents H, C₁₋₄ alkyl, C₄₋₆ cycloalkyl, or phenyl
   optionally substituted by halogen;
-(CH₂)ₐN(R¹)(R⁴); wherein
   R⁴ represents -(CH₂)ₐOR¹ or -(CH₂)ₐN(R¹)₂; and
C₁₋₄ acyl; and
CO₂R¹; wherein R1 represents H, C₁₋₄ alkyl, C₁₋₄ haloalkyl, phenyl, benzyl optionally substituted by C₁₋₃ alkyl or C₁₋₃ haloalkyl

These substituents may be positioned at any ring atom of the cyclic systems chosen for A and G. Typically, ring systems A and G of the compounds of the present invention are - independently from each other - substituted at 0, 1 or 2 position(s). Substituents of the ring systems A and G may be identical or non-identical. A and G may have more than one substituent in ortho-, meta- or para-position. If there is more than one substituent for A or G, these substitutents may again be identical or non-identical at ring system A or G.

In a still further preferred embodiment compounds of the present invention have a structure according to formula (I), wherein X represents N and Y represents S, O or NH. It is even more preferred to provide a compound of formula (I), wherein X represents N and Y represents S. Thereby, the central heteroaryl ring system of a compound of the present invention according to formula (I) represents a thiazole.

In a further preferred embodiment compounds of the present invention have a structure according to formula (I), wherein L² represents -NH(CH₂)ₐ- , -N(R¹)-, -NHC(O)NH- (urea linkage) or -N(CO)R¹-. It is particularly preferred to chose a compound of the invention, wherein L² represents -NH(CH₂)ₐ- . R¹ and a are chosen as indicated above (s. also claim 1). Particularly preferred are compounds, wherein a represents 2 or 3, respectively.

In a still further preferred embodiment compounds of the present invention have a structure according to Compound of formula (I), wherein
A represents a mono― or bicyclic aryl or heteroaryl ring system, optionally substituted;
G represents a poly- or bicyclic (hetero)aryl ring system or imidazole, pyrrole, thiophene, pyridazine, pyrazine, thiazole, or oxazole, optionally substituted;
L² represents -NH(CH₂)ₐ-, -NR¹-, -NHC(O)NH- or -NH(CO)R¹- ;
L¹ represents a chemical bond, carbonyl or -(CH₂)ₐ -.

The substitutions introduced for A and G for these preferred compounds correspond to the substitutions outlined above. It is particularly preferred to provide compounds, wherein L¹ represents a chemical bond and/or L² represents -NH(CH₂)ₐ- with a representing 1, 2 or 3.

In a still further preferred embodiment compounds of the present invention have a structure according to formula (I), wherein G is a poly- or bicyclic (hetero)aryl ring system or a C₃-C₁₂ (hetero)cycloalkyl under the provision that L¹ is a chemical bond.

Particularly preferred according to the invention are compounds of formula (I), wherein
- A represents a bicyclic aryl or heteroaryl ring system, optionally substituted, with the provision that L¹ is carbonyl;
- A represents a bicyclic aryl or heteroaryl ring system, pyrrole, furan, thiophene, pyridine, pyridazine, pyrazine, pyrimidine, pyrazole, thiazole, or oxazole, optionally substituted, or a non-aromatic C₃₋₁₂ mono- or bicyclic alkyl or heteroalkyl ring, optionally substituted,
   with the provision that L¹ is -(CH₂)ₐ- ; or
- A represents a bicyclic heteroaryl ring system, optionally substituted with the provision that A cannot be benzothiophene, or
   a substituted bicyclic aryl ring system or
   pyrrole, pyridazine, pyrazine, pyrimidine, thiazole, pyrazole, or imidazole, optionally substituted,
   with the provision that L' is a chemical bond;
G represents a mono- or bicyclic aryl or heteroaryl ring system, optionally substituted; and
L² represents -NH(CH₂)ₐ- , -NR¹-, -NHC(O)NH-, or -NH(CO)R¹-.

The substitutions introduced for A and G for these preferred compounds correspond to the substitutions outlined above. Each of alternatives for the ring systems (given above for A and G), may be substituted or not substituted. Particularly preferred are those compounds according to the invention, wherein A represents a bicyclic heteroaryl ring system with the provision that A cannot be benzothiophene, or a substituted bicyclic aryl ring system or pyrrole, pyridazine, pyrazine, pyrimidine, thiazole, pyrazole, or imidazole, whereby L¹ is a chemical bond and whereby the A representing these ring systems may be substituted.

Compounds according to the invention are particularly preferred as kinase inhibitors, particularly as Src family kinase inhibitor. Compounds may inhibit kinase activity either by competition with ATP or substrate or by binding to an allosteric site in the kinase.

The present invention provides a method of inhibiting a kinase, particularly a kinase of the Src kinase family, e.g., comprising contacting a kinase with a compound of the invention. The activity can be inhibited by at least 20%, preferably at least about 50%, more preferably at least about 60%, 70%, 80%, 90%, 95%, and most preferably at least about 98%. In one embodiment, the invention provides a method for inhibiting a kinase in vitro. In a preferred embodiment, the kinase is in vivo or ex vivo. For example, the invention provides methods for inhibiting a kinase in a cell, comprising contacting the cell with a compound of the invention, such that the activity of the kinase is inhibited. The cell may further be contacted with a composition stimulating the uptake of the compound into the cell, e,g., liposomes. In one embodiment, the invention provides a method for inhibiting a kinase in a cell of a subject, comprising administering to the subject a therapeutically effective amount of a compound of the present invention, or a formulation comprising a compound of the present invention, such that the kinase is inhibited in a cell of the subject. The subject can be one having a disease associated with a kinase, e.g., cancer. Preferred types of cancer that can be treated according to the invention include prostate cancer and breast cancer.

The therapeutic methods of the invention generally comprise administering to a subject in need thereof, a pharmaceutically effective amount of a compound of the invention, or a salt, prodrug or composition thereof. The compounds of the invention can be administered in an amount effective to inhibit the activity of a kinase. The compounds of this invention may be administered to mammals, preferably humans, either alone or, preferably, in combination with pharmaceutically acceptable carriers, excipients or diluents, in a pharmaceutical composition, according to standard pharmaceutical practice. A pharmaceutical composition comprising at least one compound according to the invention and a pharmaceutically acceptable carrier is thereby disclosed herewith.

Compounds and pharmaceutically acceptable compositions are employed for the preparation of a medicament for the treatment of a disease or a disorder or pathological condition associated with an abnormal activity or level of a kinase, in particular a Src family kinase. A variety of diseases has been associated with abnormal activity of a kinase, in particular cancer. Compounds according to the invention may be used for the preparation of a medicament for the treatment of solid tumors, e.g. lung, kidney, breast, pancreas, skin, eye, stomach, colon, bone, liver, prostate, ovarian or brain cancer. Furthermore, these compounds may also be used for therapeutical purposes for the treatment or the preparation of a medicament for the treatment of non-solid tumor forms, e.g. tumors of the blood forming system. Particularly relevant are compounds of the present invention for the treatment of various forms of leukemia and lymphoma, e.g. Hodgkin and Non-Hodgkin lymphoma, particularly for the treatment of childhood cancer.

In another preferred embodiment of the present invention compounds according to the invention are useful for polycystic kidney disease, particularly for the inhibition of a tyrosine kinase involved in the etiology of polycystic kidney disease. Another disease, which is treatable with compounds of the invention is collagen-induced arthritis, in particular with regard to the inhibition of a p38 kinase. In general, compounds of the present invention are administered for the treatment of inflammatory diseases. In particular, compounds according to the invention may act as inhibitor of e.g. p38 mitogen-activated protein (MAP) kinase, targeting the treatment of inflammatory diseases. The p38 MAP kinase enzyme regulates the production of key proinflammatory cytokines such as tumor necrosis factor-alpha (INF-alpha), interleukin-1 beta (IL-1 beta) and interleukin-6 (IL-6). Based on their mechanism of action, inhibitors of p38 MAP kinase play a role in the treatment of acute and chronic inflammatory diseases, including rheumatoid arthritis, osteoarthritis, and Crohn's disease.

Compounds according to the invention are useful for the inhibition of tyrosine kinases. Protein tyrosine kinases (PTKs) are enzymes which catalyze the phosphorylation of tyrosine residues. There are two main classes of PTKs: receptor PTKs and cellular, or non-receptor, PTKs. Of the 91 protein tyrosine kinases identified thus far, 59 are receptor tyrosine kinases and 32 are non-receptor tyrosine kinases. These enzymes are involved in cellular signaling pathways and regulate key cell functions such as proliferation, differentiation, anti-apoptotic signaling and neurite outgrowth. Unregulated activation of these enzymes, through mechanisms such as point mutations or over-expression, can lead to various forms of cancer as well as benign proliferative conditions. Indeed, more than 70% of the known oncogenes and protooncogenes involved in cancer code for PTKs. The importance of PTKs in health and disease is further underscored by the existence of aberrations in PTK signaling occurring in inflammatory diseases and diabetes. Because of their properties as tyrosine kinase inhibitors compounds according to the invention are potent modulators of the tyrosine kinase function and are useful for the treatment of pathological conditions as caused by tyrosine kinase dysfunction.

Since kinases, in particular tyrosine kinases are also involved in the etiology of inflammatory or obstructive airways diseases, agents (compounds) of the invention are consequently useful in the treatment of inflammatory or obstructive airways diseases, resulting, for example, in reduction of tissue damage, airways inflammation, bronchial hyperreactivity, remodelling or disease progression. Inflammatory or obstructive airways diseases to which the present invention is applicable include asthma of whatever type or genesis including both intrinsic (non-allergic) asthma and extrinsic (allergic) asthma, mild asthma, moderate asthma, severe asthma, bronchitic asthma, exercise-induced asthma, occupational asthma and asthma induced following bacterial infection.

Kinase inhibitors (compounds) according to the invention may be used for the treatment of allograft rejection, allergic reactions, immunosuppression (particularly, by inhibition of kinases (e.g. tyrosine kinases), which are involved in the activation of cells of the immune system (e.g. B- or T-Lymphocytes) and for the treatment of osteoporosis and related bone diseases, including osteolytic bone metastases (target: c-Src, a member of the Src kinase family). Compounds according to the invention for the treatment of osteoporosis are based on (i) their potential as Src homology (SH)-2 inhibitors incorporating non-hydrolyzable phosphotyrosine mimics and exhibiting molecular recognition and bone-targeting properties; and (ii) on their potential as ATP-based Src kinase inhibitors incorporating bone-targeting moieties. Thereby compounds according to the invention may differ mechanistically by virtue of blocking Src-dependent non-catalytic or catalytic activities in osteoclasts.

All of the aforementioned diseases represent "diseases associated with a kinase".

The compounds can be administered orally or parenterally, including the intravenous, intramuscular, intraperitoneal, subcutaneous, rectal and topical routes of administration.

Toxicity and therapeutic efficacy of the compounds can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Compounds which exhibit large therapeutic indices are preferred. While compounds that exhibit toxic side effects may be used, care should be taken to design a delivery system that targets such reagents to the site of affected tissue in order to minimize potential damage to normal cells and, thereby, reduce side effects.

Data obtained from cell culture assays and animal studies can be used in formulating a range of dosage for use in humans. The dosage of such compounds lies preferably within a range of circulating concentrations that include the ED₅₀ with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC₅₀ (i.e., the concentration of the test compound which achieves a half- maximal inhibition of activity) as determined in cell culture. Such information can be used to more accurately determine useful doses in humans. The compounds of the invention have an IC₅₀ less than 10 µM as determined by the biochemical or cellular assay described herein. Some compounds of the invention are effective at concentrations of 10 nM, 100 nM, or 1 µM. Based on these numbers, it is possible to derive an appropriate dosage for administration to subjects. Formation of prodrugs is well known in the art in order to enhance the properties of the parent compound, Such properties include solubility, absorption, biostability and release time (see "Pharmaceutical Dosage Form and Drug Delivery Systems" (Sixth Edition), edited by Ansel et al., publ. by Williams & Wilkins, pgs. 27-29, (1995)). Commonly used prodrugs of the disclosed compounds can be designed to take advantage of the major drug biotransformation reactions and are also to be considered within the scope of the invention. Major drug biotransformation reactions include N-dealkylation, O-dealkylation, aliphatic hydroxylation, aromatic hydroxylation, N-oxidation, S-oxidation, deamination, hydrolysis reactions, glucuronidation, sulfation and acetylation (see Goodman and Gilman's The Pharmacological Basis of Therapeutics (Ninth Edition), editor Molinoff et al., publ. by McGraw-Hill, pages 11-13, (1996)). The pharmaceutical compositions can be prepared so that they may be administered orally, dermally, parenterally, nasally, ophthalmically, otically, sublingually, rectally or vaginally. Dermal administration includes topical application or transdermal administration. Parenteral administration includes intravenous, intraarticular, intramuscular, intraperitoneal, and subcutaneous injections, as well as use of infusion techniques. One or more compounds of the invention may be present in association with one or more non-toxic pharmaceutically acceptable ingredients and. optionally, other active anti-proliferative agents, to form the pharmaceutical composition. These compositions can be prepared by applying known techniques in the art such as those taught in Remington's Pharmaceutical Sciences (Fourteenth Edition), Managing Editor, John E. Hoover, Mack Publishing Co., (1970) or Pharmaceutical Dosage Form and Dug Delivery Systems (Sixth Edition), edited by Ansel et al., publ. by Williams & Wilkins, (1995).

As indicated above, pharmaceutical compositions containing a compound of the invention may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically acceptable preparations. Tablets contain the active ingredient in admixture with non-toxic pharmaceutically acceptable excipients which are suitable for the manufacture of tablets. These excipients may be, for example, inert diluents, such as calcium carbonate, sodium carbonate, lactose, calcium phosphate or sodium phosphate; granulating and disintegrating agents, for example, microcrystalline cellulose, sodium crosscarmellose, corn starch, or alginic acid; binding agents, for example starch, gelatin, polyvinyl-pyrrolidone or acacia; and lubricating agents, for example, magnesium stearate, stearic acid or talc, The tablets may be uncoated or they may be coated by known techniques to mask the unpleasant taste of the drug or delay disintegration and absorption in the gastrointestinal tract and thereby provide a sustained action over a longer period. For example, a water soluble taste masking material such as hydroxypropylmethyl-cellulose or hydroxypropylcellulose, or a time delay material such as ethyl cellulose, cellulose acetate buryrate may be employed.

Formulations for oral use may also be presented as hard gelatin capsules wherein the active ingredient is mixed with an inert solid diluent, for example, calcium carbonate, calcium phosphate or kaolin, oz as soft gelatin capsules wherein the active ingredient is mixed with water soluble carrier such as polyethyleneglycol or an oil medium, for example peanut oil, liquid paraffin, or olive oil.

Aqueous suspensions contain the active material in admixture with excipients suitable for the manufacture of aqueous suspensions. Such excipients are suspending agents, for example sodium carboxymethylcellulose, methylcellulose, hydroxypropylmethyl- cellulose, sodium alginate, polyvinyl-pyrrolidone, gum tragacanth and gum acacia; dispersing or wetting agents may be a naturally occurring phosphatide, for example lecithin; or condensation products of an alkylene oxide with fatty acids, for example polyoxyethylene stearate; or condensation products of ethylene oxide with long chain aliphatic alcohols, for example heptadecaethylene-oxycetanol; or condensation products of ethylene oxide with partial esters derived from fatty acids and a hexitol such as polyoxyethylene sorbitol monooleate; or condensation products of ethylene oxide with partial esters derived from fatty acids and hexitol anhydrides, for example polyethylene sorbitan monooleate. The aqueous suspensions may also contain one or more preservatives, for example ethyl or n-propyl p-hydroxybenzoate, one or more coloring agents, one or more flavoring agents, and one or more sweetening agents, such as sucrose, saccharin or aspartame.

Oily suspensions may be formulated by suspending the active ingredient in a vegetable oil, for example arachis oil, olive oil, sesame oil or coconut oil, or in mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as butylated hydroxyanisol or alpha-tocopherol.

Dispersible powders and granules suitable for preparation of an aqueous suspension by the addition of water provide the compound of the invention in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present. These compositions may be preserved by the addition of an anti-oxidant such as ascorbic acid.

Pharmaceutical compositions of the invention may also be in the form of an oil-in- water emulsions. The oily phase may be a vegetable oil, for example olive oil or arachis oil, or a mineral oil, for example liquid paraffin or mixtures of these. Suitable emulsifying agents may be naturally occurring phosphatides, for example soy bean lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening, flavouring agents, preservatives and antioxidants.

Syrups and elixirs may be formulated with sweetening agents, for example glycerol, propylene glycol, sorbitol or sucrose. Such formulations may also contain a demulcent, a preservative, flavoring and coloring agents and antioxidant.

Pharmaceutical compositions may be in the form of a sterile injectable aqueous solutions. Among the acceptable vehicles and solvents that may be employed are water, Ringer's solution and isotonic sodium chloride solution.

Sterile injectable preparation may also be a sterile injectable oil-in-water microemulsion where the compound of the invention is dissolved in the oily phase. For example, the active ingredient may be first dissolved in a mixture of soybean oil and lecithin. The oil solution is then introduced into a water and glycerol mixture and processed to form a microemulation.

The injectable solutions or microemulsions may be introduced into a patient's blood stream by local bolus injection. Alternatively, it may be advantageous to administer the solution or microemulsion in such a way as to maintain a constant circulating concentration of the active compound. In order to maintain such a constant concentration, a continuous intravenous delivery device may be utilized. An example of such a device is the Deltec CADD-PLUS°° model 5400 intravenous pump.

The pharmaceutical compositions may be in the form of a sterile injectable aqueous or oleagenous suspension for intramuscular and subcutaneous administration. This suspension may be formulated according to the known art using those suitable dispersing or wetting agents and suspending agents which have been mentioned above. The sterile injectable preparation may also be a sterile injectable solution or suspension in a nontoxic parenterally acceptable diluent or solvent, for example as a solution in 1,3-butane diol. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Compounds of the invention may also be administered in the form of a suppository for rectal administration of the drug. These compositions can be prepared by mixing the drug with a suitable non-irritating excipient which is solid at ordinary temperatures but liquid at the rectal temperature and will therefore melt in the rectum to release the drug. Such materials include cocoa butter, glycerinated gelatin, hydrogenated vegetable oils, mixtures of polyethylene glycols of various molecular weights and fatty acid esters of polyethylene glycol.

For topical use, creams, ointments, jellies, solutions or suspensions, etc., containing the compound of the invention can be employed. For purposes of this application, topical application shall include mouth washes and gargles.

The compounds for the present invention can be administered in intranasal form via topical use of suitable intranasal vehicles and delivery devices, or via transdermal routes, using those forms of transdermal skin patches well known to those of ordinary skill in the art. To be administered in the form of a transdermal delivery system, the dosage administration will preferably be continuous rather than intermittent throughout the dosage regimen.

The compounds of the invention may also be co-administered with other well known therapeutic agents that are selected for their particular usefulness against the condition that is being treated. The compounds may be administered simultaneously or sequentially. For example, the active compounds may be useful in combination with known anti-cancer and cytotoxic agents. Similarly, the active compounds may be useful in combination with agents that are-effective in the treatment and prevention of osteoporosis, inflammation, neurofibromatosis, restenosis, and viral infections. The active compounds may also be useful in combination with inhibitors of other components of signaling pathways of cell surface growth factor receptors. Drugs that can be co-administered to a subject being treated with a compound of the invention include antineoplastic agents selected from vinca alkaloids, epipodophyllotoxins, anthracycline antibiotics, actinomycin D, plicamycin, puromycin, gramicidin D, taxol, colchicine, cytochalasin B, emetine, maytansine, or amsacrine. Methods for the safe and effective administration of most of these chemotherapeutic agents are known to those skilled in the art. In addition, their administration is described in the standard literature, For example, the administration of many of the chemotherapeutic agents is described in the "Physicians' Desk Reference" (PDR), 1996 edition (Medical Economics Company, Montvale, N.J. 07645-1742, USA).

Radiation therapy, including x-rays or gamma rays which are delivered from either an externally applied beam or by implantation of tiny radioactive sources, may also be used in combination with a compound of the invention to treat a disease, e.g., cancer.

When a composition according to this invention is administered into a human subject, the daily dosage will normally be determined by the prescribing physician with the dosage generally varying according to the age, weight, and response of the individual patient, as well as the severity of the patient's symptoms.

In one embodiment, a compound of the invention, materials and/or reagents required for administering the compounds of the invention may be assembled together in a kit. When the components of the kit are provided in one or more liquid solutions, the liquid. solution preferably is an aqueous solution, with a sterile aqueous solution being particularly preferred.

The kit may further comprise one or more other drugs, e.g., a chemo- or radiotherapeutic agent, These normally will be a separate formulation, but may be formulated into a single pharmaceutically acceptable composition. The container means may itself be geared for administration, such as an inhalant, syringe, pipette, eye dropper, or other such like apparatus, from which the formulation may be applied to an infected area of the body, such as the lungs, or injected into an animal, or even applied to and mixed with the other components of the kit. The compositions of these kits also may be provided in dried or lyophilized forms. When reagents or components are provided as a dried form, reconstitution generally is by the addition of a suitable solvent. It is envisioned that the solvent also may be provided in another container means. The kits of the invention may also include an instruction sheet defining administration of the agent. Kits may also comprise a compound of the invention, labeled for detecting kinases. The kits of the present invention also will typically include a means for containing the vials in close confinement for commercial sale such as, e.g., injection or blow-molded plastic containers into which the desired vials are retained. Irrespective of the number or type of containers, the kits of the invention also may comprise, or be packaged with a separate instrument for assisting with the injection/administration or placement of the ultimate complex composition within the body of an animal. Such an instrument may be an inhalant, syringe, pipette, forceps, measured spoon, eye dropper or any such medically approved delivery vehicle. Other instrumentation includes devices that permit the reading or monitoring of reactions or amounts of compounds or polypeptides.

The compounds of the present invention may be used as component of a method of treatment of disease associated with a kinase. Hereby, a compound or a composition of the present invention is administered by a method disclosed above to the patient (animal or human). Dosage requirements are dependent e.g. upon the age, the body weight, gender, the method of administration (e.g. orally or parenterally) and the severity of the disease.

### General Method for the Preparation of Compounds of Thiazoles of Formula I

Thiazoles of Formula I, wherein A, L¹, J, L² and G are as described in any of claims 1-14 are 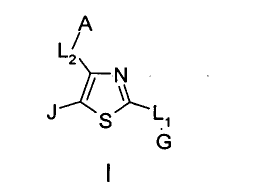 prepared by the general method described below, according to methods described below or according to methods commonly employed in the art.

Compounds of Formula I are prepared according to
Scheme 1, whereby halo ketone III, wherein X is Cl, Br, I, or other leaving group commonly employed in the art, is treated with thioamide VI in a polar solvent, such as an alcoholic solvent, at a temperature between 40 - 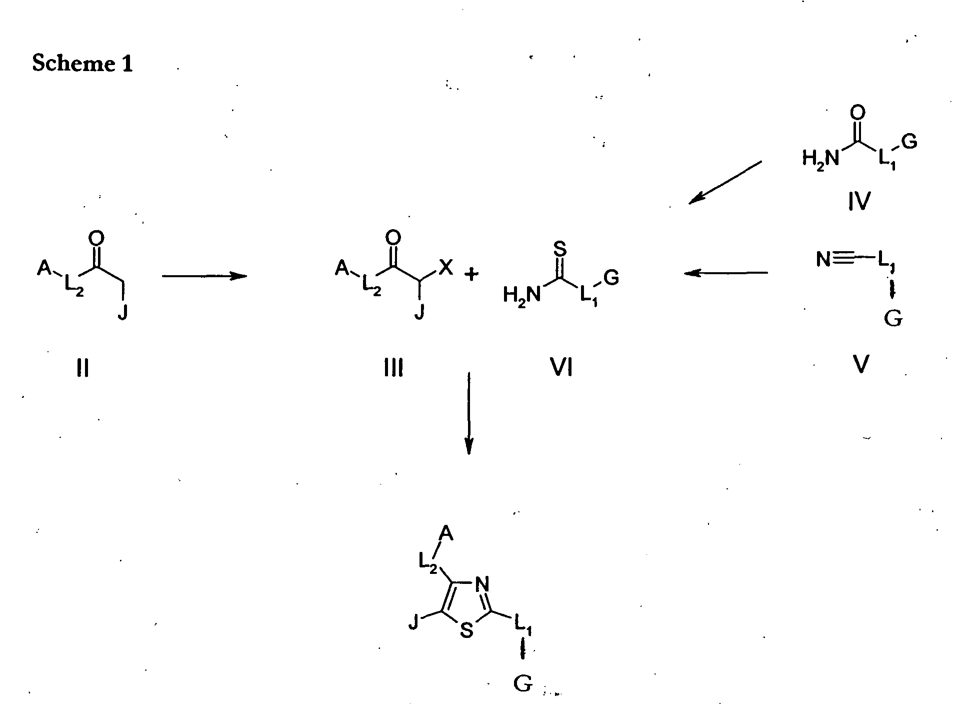 120 °C. Preferably, the polar solvent is an alcohol such as ethanol, 1-propanol, or 2-propanol. Halo ketones III are commercially available or may be prepared using an electrophilic halogen reagent such as bromine, N-chlorosuccinimide, N-bromosuccinimide, or phenyltrimethylammonium tribromide using the general methods or as outlined in the specific examples described below or other methods commonly employed in the art. Alternatively, the corresponding alphahydroxy ketone can be converted into III using standard conditions employed in the art to convert an alcohol functionality into a halogen or other leaving group commonly employed in the art. Ketones III are commercially available or are prepared according to methods commonly employed in the art. Thioamide VI can be prepared from nitrile V upon treatment with hydrogen sulphide. Alternatively, VI can be prepared from amide IV upon treatment with Lawessons reagent or P₄S₁₀. Nitriles V are commercially available or can be prepared according the methods commonly employed in the art. Amides are commercially available or they can be prepared by methods commonly employed in the art to prepare amide functionality from carboxylic acid functionality, whereby the requisite carboxylic acid is commercially available or can be prepared by methods commonly employed in the art.

Compounds of Formula I, when L¹ is N, are prepared according to Scheme 2. Amines VII 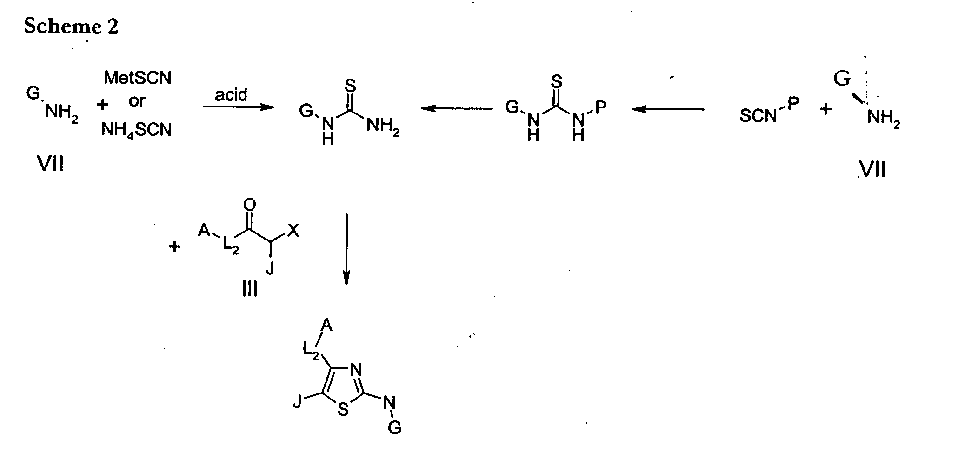 are treated with ammonium thiocyanates or with alkali metal thiocyanates in the presence of acid in an inert organic solvent, such as bromobenzene, chlorobenzene, xylene, toluene, THF, or dioxane at a temperature between 60 - 250 °C. Alternatively, the required thioureas can be prepared from the amine VII upon treatment with an isothiocyanate bearing a suitable protecting group like benzoyl or Fmoc or other protecting group commonly employed in the art followed by deprotection using the general methods described below or other methods commonly employed in the art.

The present invention is further illustrated by the following examples which should not be construed as limiting in any way. The contents of all cited references (including literature references, issued patents, published patent applications as cited throughout this application) are hereby expressly incorporated by reference.

### Examples

All reagents are commercially available unless otherwise specified. Reagents were used as received unless otherwise specified. Proton NMR data is reported downfield from TMS; coupling constants are in hertz. NMR data are in agreement with the structure of all prepared compounds.

### 1. Procedure for the preparation of the thioureas:

### 1.1 Method A:

### Preparation of Pyrimidin-2-yl-thiourea:

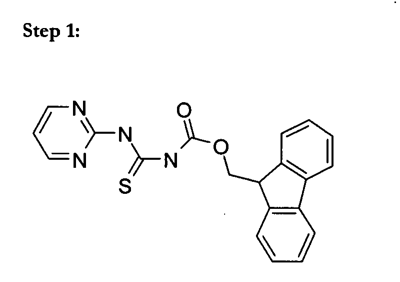

To a stirred solution of 2-aminopyrimidine (0.285 g, 3.0 mmol) in toluene (10.0 ml) was added Fmoc-NCS (0.90 g, 3.2 mmol) and the reaction was heated to reflux for four hours. The reaction was cooled to room temperature and the product filtered off as a beige solid (1.027 g, 92%) LCMS 377 (M+H)⁺; δ_{H} (300 MHz) (DMSO d₆) 4.30 (1 H, t, J = 8.9), 4.44 (2 H, d,J = 8.0), 7.27-7.45 (5 H, m), 7.77 (2 H, d, J = 8.7), 7.91 (2 H, d,J = 8.4), 8.72 (2 H, d, J = 5.8), 11.82 (1 H, s), 12.70 (1 H, s).

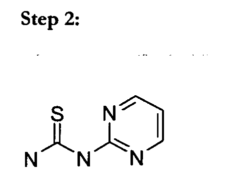

To a stirred solution of NFmoc protected thiourea (0,50 g, 1.3 mmol) in DCM (10.0 ml) was added piperidine (2.0 ml) and the reaction stirred. After three hours the product was isolated by filtration and washed with DCM to give a white solid (0.164 g, 82%), LCMS 154 [M]⁺.

### 1.2 Method B:

### (a) Preparation of [2-(1H-Indol-3-yl)-ethyl]-thiourea:

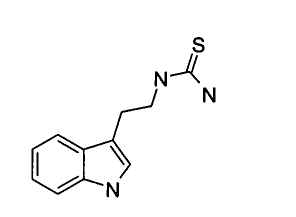

Tryptamine hydrochloride (1.00 g, 5.08 mmol) and potassium thiocyanate (0.741 g, 7.63 mmol) were added to dioxane (20 ml). The suspension was heated to reflux for 48 hours under an atmosphere of argon. The reaction was cooled to room temperature and was filtered over a plug of silica and washed with ethyl acetate. The filtrate was concentrated under reduced pressure to give the product as a beige solid (1.09 g, 4.95 mmol, 97%). δ_{H} (300 MHz) (DMSO d₆ 2.89 (2 H, t, J = 7), 3.66 (2 H, s, br), 6.94-7.00 (2 H, m), 7.07 (1 H, dt, J = 8, J = 1.3), 7.16 (1 H, s), 7.34 (1 H, d, J = 8.0), 7.60 (2 H, m), 10.81 (1 H, s).

### (b) Preparation of (1H-Benzoimidazol-2-yl)-thiourea:

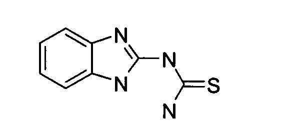

1H-Benzoimidazol-2-ylamine (1.00 g, 7.51 mmol) was dissolved in dioxane (15 ml). Potassium thiocyanate (1.08 g, 11.1 mmol) was added and HCl (4 M in dioxane, 3.0 ml, 12 mmol). The mixture was heated to reflux for 48 hours, cooled to room temperature and filtered over a plug of silica and washed with ethanol. The filtrate was concentrated under reduced pressure to give the product as a brown solid (1.4 g, 7.3 mmol, 97%). δ_{H} (300 MHz) (DMSO d₆) 7.17-7.23 (2 H, m), 7.33-7.39 (2 H, m), 8.44 (2 H, s).

### (c) Preparation of Furan-2-ylmethyl-thiourea:

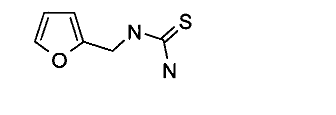

C-Furan-2-yl-methylamine (1.00 g, 10.3 mmol) was dissolved in dioxane (15 ml). Potassium thiocyanate (1.50 g, 15.4 mmol) was added and HCl (4 M in dioxane, 2.6 ml, 10 mmol). The mixture was heated to reflux for 48 hours, cooled to room temperature and filtered over a plug of silica and washed with ethyl acetate. The filtrate was concentrated under reduced pressure to give the product as a brown solid (1.74 g, 10.0 mmol, 98%). δ_{H} (300 MHz) (DMSO d₆ 4.60-4.65 (2 H, m), 6.28-6.29 (1 H, m), 6.39-6.41 (1 H, m), 7.08 (1 H, s, br), 7.59-7.60 (m, 1 H), 7.92 (1 H, s, br).

### (d) Preparation of Piperidine-1-carbothioic acid amide:

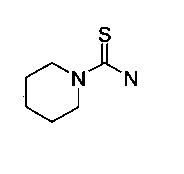

Piperidine (1.00 g, 11.7 mmol) was dissolved in dioxane (15 ml). Potassium thiocyanate (1.71 g, 17.6 mmol) was added and HCl (4 M in dioxane, 2.9 ml, 12 mmol). The mixture was heated to reflux for 48 hours, cooled to room temperature and filtered over a plug of silica and washed with ethyl acetate. The filtrate was concentrated under reduced pressure to give the product as a pale brown solid (0.238 g, 1.65 mmol, 14%). δ_{H} (300 MHz) (DMSO d₆) 1.41-1.66 (6 H, m), 3.69- 3.71 (4 H, m), 7.23 (2 H, s).

### Preparation of compound:

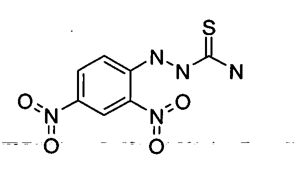

(2,4-Dinitro-phenyl)-hydrazine (1.00 g, 5.05 mmol) was dissolved in dioxane (15 ml). Potassium thiocyanate (0.736 g, 7.57 mmol) was added and HCl (4 M in dioxane, 1.3 ml, 5.1 mmol). The mixture was heated to reflux for 48 hours, cooled to room temperature and filtered over a plug of silica and washed with ethyl acetate. The filtrate was concentrated under reduced pressure to give the product as a red brown solid (1.26 g, 4.90 mmol, 97%). δ_{H} (300 MHz) (DMSO d₆) 7.09 (1 H, d, J = 9.3), 7.88 (1 H, s, br), 8.10 (1 H, s, br), 8.43 (1 H, dd, J = 2.7, J = 9.3), 8.83 (1 H, d, J = 2.7), 9.90 (1 H, s), 10.18 (1 H, s).

### Preparation of 2-Bromo-1-(3,4,5-trimethoxy-phenyl)-ethanone:

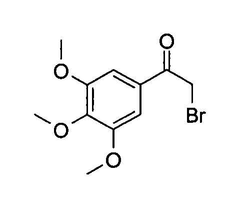

To a 0 °C cold solution of 1-(3,4,5-trimethoxy-phenyl)-ethanone (3.00 g, 14.3 mmol) in chloroform/diethyl ether (100 ml, 2:1) was slowly added bromine (2.3 g, 14 mmol) in chloroform (15 ml) dropwise. The mixture was warmed to room temperature and stirred for 1 hour. The reaction was poured into water and extracted with chloroform. The combined organic phases were washed with saturated NaHCO₃ and brine, dried with Na₂SO₄, filtered and concentrated under reduced pressure. Purification by flash column chromatography (eluent 70% heptane:ethyl acetate) gave the required product as a colorless solid (2.95 g, 10.2 mmol, 72%). δ_{H} (300 MHz) (CDCl₃) 3.94 (6 H, s), 3.96 (3 H, s), 4.42 (2 H, s), 7.26 (2 H, s).

### Preparation of [4-(2,5-Dimethoxy-phenyl)-thiazol-2-yl]-[2-(1H-indol-3-yl)-ethyl]-amine hydrobromide:

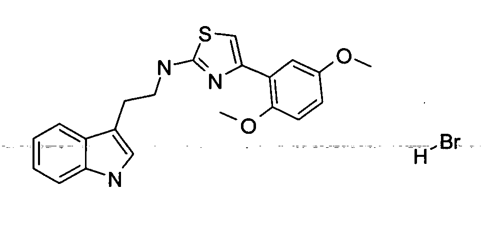

[2-(1H-Indol-3-yl)-ethyl]-thiourea (79 mg, 0.36 mmol) and 2-bromo-1-(2,5-dimethoxy-phenyl)-ethanone (93 mg, 0.36 mmol) were dissolved in abs. methanol (5 ml). The mixture was heated to reflux for two hours under an atmosphere of argon, cooled to 0 °C and the solid was filtered off as a colorless solid and was washed with cold methanol (122 mg, 0.265 mmol, 74%). δ_{H} (300 MHz) (DMSO d₆) 3.07 (2 H, t, J = 7.1), 3.68 (2 H, t, J = 7.1), 3.82 (3 H, s), 3.86 (3 H, s), 6.64 (1 H, dd, J = 2, J = 8), 6.70 (1 H, d, J = 2), 6.97 (1 H, s), 6.98-7.03 (1 H, m), 7.06- 7.12 (1 H, m), 7.24 (1 H, d, J = 2), 7.35-7.38 (1 H, m), 7.53 (1 H, d, J = 8), 7.59 (1 H, d, J = 8), 10.91 (1 H, s).

### Preparation of [2-(1H-Indol-3-yl)-ethyl]-[4-(4-pyrrolidin-1-yl-phenyl)-thiazol-2-yl]-amine hydrobromide:

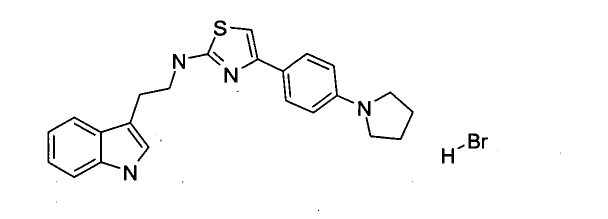

[2-(1H-Indol-3-yl)-ethyl]-thiourea (61 mg, 0.28 mmol) and 2-bromo-1-(4-pyrrolidin-1-yl-phenyl)-ethanone (75 mg, 0.28 mmol) were dissolved in abs. methanol (5 ml). The mixture was heated to reflux for two hours under an atmosphere of argon, cooled to 0 °C and the solid was filtered off as a green yellow solid and was washed with cold methanol (118 mg, 0.219 mmol, 79%). δ_{H} (300 MHz) (DMSO d₆) 1.95-1.99 (4 H, m), 3.08 (2 H, t, J = 7.1), 3.25-3.29 (4 H, m), 3.72 (2 H, t, J = 7.1), 6.60 (1 H, d, J = 8.8), 6.83 (1 H, s), 6.99-7.04 (1 H, m), 7.06-7.12 (1 H, m), 7.25 (1 H, d, J = 2.2), 7.36 (1 H, d, J = 8.0), 7.50 (1 H, d,J = 8.4), 7.61 (1 H, d, J = 7.5), 10.91 (1 H, s).

### Preparation of (8H-Indeno[1,2-d]thiazol-2-yl)-[2-(1H-indol-3-yl]-ethyl]-amine hydrobromide:

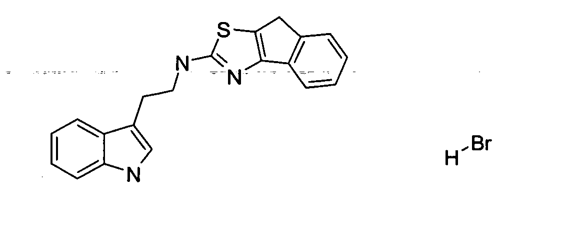

[2-(1H-Indol-3-yl)-ethyl]-thiourea (45 mg, 0.21 mmol) and 2-bromo-indan-1-one (43 mg, 0.21 mmol) were dissolved in abs. methanol (5 ml). The mixture was heated to reflux for two hours under an atmosphere of argon, cooled to 0 °C and the solid was filtered off as a colorless solid and was washed with cold methanol (27 mg, 0.065 mmol, 32%). δ_{H} (300 MHz) (DMSO d₆) 3.11 (2 H, t, J = 7), 3.74 (2 H, t, J = 7), 3.80 (2 H, s), 6.99-7.04 (1 H, m), 7.07-7.12 (1 H, m), 7.24-7.29 (2 H, m), 7.35-7.40 (2 H, m), 7.55 (1 H, d, J = 7.1), 7.61-7.65 (2 H, m), 10.92 (1 H, s).

### Preparation of [2-(1H-Indol-3-yl)-ethyl]-(5-methyl-4-phenyl-thiazol-2-yl)-amine hydrobromide:

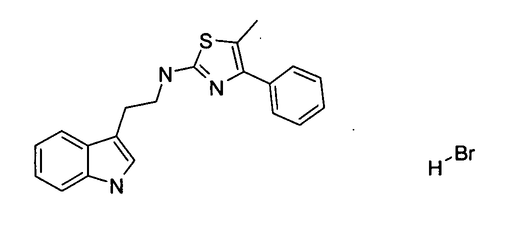

[2-(1H-Indol-3-yl)-ethyl]-thiourea (45 mg, 0.21 mmol) and 2-bromo-1-phenyl-propan-1-one (44 mg, 0.21 mmol) were dissolved in abs. methanol (4 ml). The mixture was heated to reflux for two hours under an atmosphere of argon and cooled to room temperature. The solvent was removed under reduced pressure. The residue was added to ethyl acetate/petrol ether 1:1 and heated to reflux, cooled to 0 °C and the solid was filtered off as a colorless solid and was washed with ethyl acetate/petrol ether 1:1 (80 mg, 0.19 mmol, 94%). δ_{H} (300 MHz) (DMSO d₆) 2.23 (3 H, s), 3.07 (2 H, t, J = 7.1), 3.73 (2 H, t, J = 7.1), 6.99-7.04 (1 H, m), 7.07-7.12 (1 H, m), 7.25 (1 H, d, J = 1.8), 7.37 (1 H, d, J = 8.0), 7.44-7.56 (5 H, m), 7.56 (1 H, d, J = 7.5), 9.79 (1 H, s, br), 10.95 (1 H, s).

### Preparation of 2-[2-(1H-Indol-3-yl)-ethylamino]-thiazole-4-carboxylic acid hydrobromide:

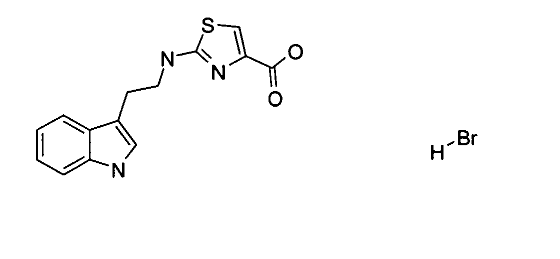

[2-(1H-Indol-3-yl)-ethyl]-thiourea (39 mg, 0.18 mmol) and bromopyruvic acid (30 mg, 0.18 mmol) were dissolved in abs. methanol (2 ml). The mixture was heated to reflux for 1.5 hours under an atmosphere of argon and the solvent removed under reduced pressure. The residue was added to ethyl acetate/petrol ether 1:1 and heated to reflux, cooled to room temperature and the solid was filtered off as a colorless solid and washed with ethyl acetate/petrol ether 1:1 (61 mg, 0.17 mmol, 93%). δ_{H} (300 MHz) (DMSO d₆) 3.02 (2 H, t, J = 7.1), 3.68 (2 H, t, J = 7.1), 6.96-7.02 (1 H, m), 7.05-7.10 (1 H, m), 7.22 (1 H, d, J = 2.2), 7.35 (1 H, d, J = 8.0), 7.58 (1 H, d, J = 8.0), 7.61 (1 H, s), 10.90 (1 H, s).

### Preparation of (4-tert-Butyl-thiazol-2-yl-2-[1H-indol-3-yl)-ethyl]-amine hydrobromide:

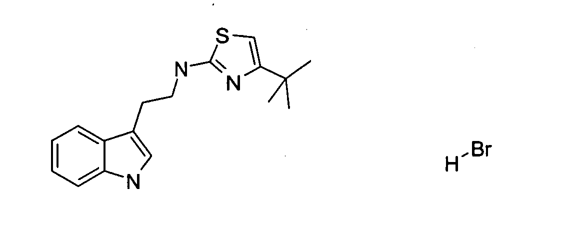

[2-(1H-Indol-3-yl)-ethyl]-thiourea (34 mg, 0.16 mmol) and 1-bromo-3,3-dimethyl-butan-2-one (28 mg, 0.21 mmol) were dissolved in abs. methanol (2 ml). The mixture was heated to reflux for two hours under an atmosphere of argon and the solvent removed under reduced pressure. The residue was added to ethyl acetate/petrol ether 1:1 and heated to reflux, cooled to room temperature and the solid was filtered off as a colorless solid and washed with ethyl acetate/petrol ether 1:1 (49 mg, 0.13 mmol, 83%). δ_{H} (300 MHz) (DMSO d₆) 1.24 (9 H, s), 3.06 (2 H, t, J = 7.1), 3.67 (2 H, s, br), 6.48 (1 H, s), 6.97-7.02 (1 H, m), 7.05-7.11 (1 H, m), 7.25 (1 H, d, J = 2.2), 7.36 (1 H, d, J = 8.0), 7.57 (1 H, d, J = 7.5), 10.93 (1 H, s).

### Preparation of 2-Benzo[b]thiophen-3-yl-4-(2,5 dimethoxy-phenyl)-thiazole:

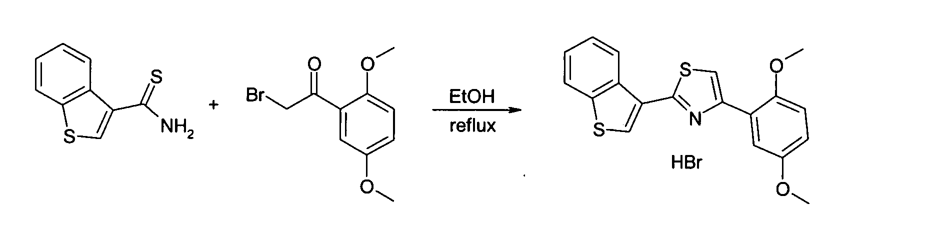

To a solution of 1-Benzothiophene-3-carbothioamide (1.93 g, 10 mmol) in abs. ethanol (100 mL) was added 2-bromo-1-(2,5-dimethoxy-phenyl)-ethanone (2.59 g, 10 mmol) and the solution refluxed overnight under an Ar atmosphere. After cooling in ice water, the solid was filtered, sequentially washed with ethanol and hexane, and then dried to afford 2.82 g (80 %) of a pale yellow solid: LCMS 354 (M+H)⁺.

### Preparation of (2-Benzo[1,3]dioxol-5-yl-5-methyl-thiazol-4-yl)-phenyl-amine:

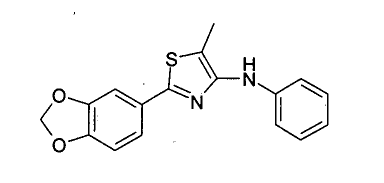

A homogenous mixture of 1,3-Benzodioxole-5-carbothioamide (1.81 g , 10 mmol) and 2-bromo-N-phenylpropionamide (2.28 g, 10 mmol) was melted at 110°C for 20 h. The melt was suspended in CH₂Cl₂ (65 mL) and free-based with triethylamine (1.4 mL). The suspension was filtered to remove starting material and the filtrate purified by silica gel chromatography to give the product as light yellow crystals in 5 % yield (155 mg, 0.50 mmol): LCMS 311 (M+H)⁺.

### Preparation of 2-(4-Isopropoxy-thiazol-2-yl-)-1-methyl-1H-benzoimidazole:

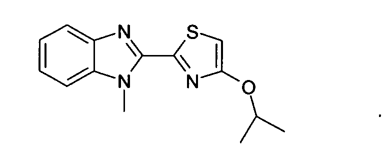

A suspension of 1-methyl-1H-benzimidazole-2-carbothioamide (1.91 g, 10 mmol) and N-(bromoacetyl)-3,5-dichloroaniline (1.42 g, 5 mmol) in isopropanol (30 mL) was refluxed for 16 h. The solvent was then boiled off and the solid residue suspended in CH₂Cl₂ (30 mL). The crude suspension was free-based with triethylamine (1.39 mL), and filtered. Purification by silica gel chromato-graphy gave the product as a clear oil in 25% yield (684 mg, 2.50 mmol): LCMS 274 (M+H)⁺.

### Preparation of 4-Benzyloxy-2-(2H-chromen-3-yl)-thiazole:

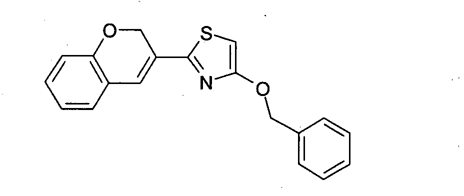

2H-Chromene-3-carbothioamide (1.91 g, 10 mmol) was heated in neat benzyl bromoacetate (11.45 g, 50 mmol) at 90 °C for 1h. The reaction was diluted with CH₂Cl₂ (40 mL) and quenched with triethylamine (2.79 mL). This was purified by silica gel chromatography to give the product as an orange solid in 5 % yield (160.70 mg): LCMS 322 (M+H)⁺.

### Preparation of 1-Benzo[b]thiophen-2-yl-3-[4-(2,5-dimethoxy-phenyl]-thiazol-2-yl]-urea:

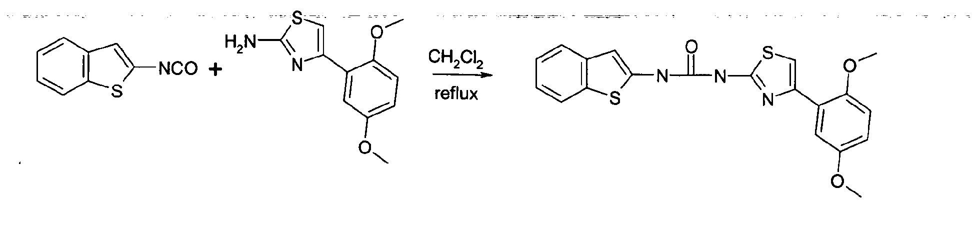

Thiourea (76.1 mg, 1.0 mmol) and 2-bromo-1-(2,5-dimethoxy-phenyl)-ethanone (259.1 mg, 1.0 mmol) were dissolved in abs. CH₂Cl₂ (15 ml). The mixture was heated to reflux for two hours under an atmosphere of argon, cooled to 0 °C and the solid was filtered off as a colorless solid and was washed with cold methanol. The product was suspended in CH₂Cl₂ (10 mL) and free-based with triethylamine (0.15 mL) to give the 4-(2,5-Dimethoxy-phenyl)-thiazol-2-ylamine (165.4 mg, 0.7 mmol, 70%).
4-(2,5-Dimethoxy-phenyl)-thiazol-2-ylamine (118.1 mg, 0.5 mmol) and 1-benzothiophen-3-yl isocyanate (87.6 mg , 0.5 mmol) were dissolved in abs. CH₂Cl₂ and refluxed overnight. This was purified by silica gel chromatography to give the product as an pale yellow solid in 50 % yield (102.9 mg): LCMS 412 (M+H)⁺.

### Preparation of 4-(2,5-Dimethoxy-phenyl)-thiazol-2-ylamine; compound with 1-benzofuran-2-yl-ethanone

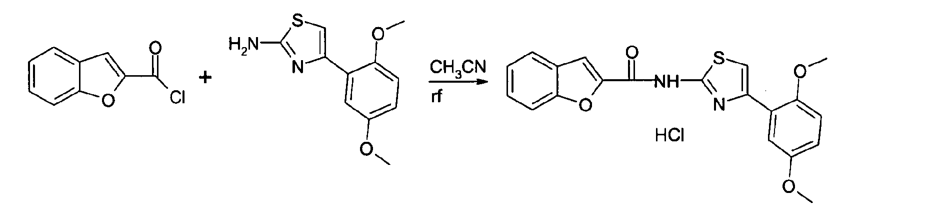

To a stirred solution of free based 4-(2,5-Dimethoxy-phenyl)-thiazol-2-ylamine (236.2 mg, 1.0 mmol) in acetonitrile (6.0 ml) was added pyridine (0.15 g, 2.00 mmol) and 1-Benzofuran-2-carbonyl chloride (198.6 mg, 1.1 mmol) and the solution was heated to 70°C for 1 hour. After cooling the product was isolated by filtration as a yellow solid (35 % yield, 82.65 mg, 0.35 mmol): LCMS 237 (M+H)⁺.

### Abbreviations and Acronyms

When the following abbreviations are used throughout the disclosure, they have the following meaning:
- A: Angstrom
- AcOH: acetic acid
- amu: atomic mass units
- Anal. Calcd: analysis calculated
- Ar: argon
- BSA: bovine serum albumin
- n-BuLi: butyllithium
- CDCl₃: chloroform-d
- CD₃OD: methanol-d₄
- CHCl₃: chloroform
- CH₂Cl₂: methylene chloride
- CH₃CN: acetonitrile
- CI: chemical ionization (in mass spectrometry)
- CuI: copper iodide
- Cs₂CO₃: cesium carbonate
- CPM: counts per minute
- DMF: dimethylformamide
- DMSO: dimethylsulfoxide
- DMSO-d₆: dimethylsulfoxide-d₆
- EDCI EI: electron impact (in mass spectrometry)
- EPA: Environmental Protection Agency (as in EPA vial)
- ES: electrospray ionization (in mass spectrometry)
- Et₃N: triethylamine
- EtOAc: ethyl acetate
- Et₂O: diethyl ether
- EtOH: ethanol
- ETPB: ethyltriphenylphosphonium bromide
- g: gram
- GCEI: gas chromatography - electron impact mass spectrometry
- GCMS: gas chromatography / mass spectrometry
- h: hour(s)
- H₂: hydrogen gas
- HBr: hydrogen bromide
- HCI: hydrochloric acid
- ¹H NMR: proton nuclear magnetic resonance
- HEPES: 4-(2-Hydxoxyethyl)piperazine-1-ethanesulfonic acid
- HOAc: acetic acid
- HOAt: n-hydroxyazatriazole
- HPLC: high performance liquid chromatography
- H₂S: hydrogen sulfide
- Hz: hertz
- KHMDS: potassium bis(trimethylsilyl) amide
- KOH: potassium hydroxide
- L: liter(s)
- LCMS: liquid chromatography / mass spectroscopy
- LDA: lithium diisopropyl amide
- M: molar
- MCPBA: m-chloroperbenzoic acid
- MeCN: acetonitrile
- MeOH: methanol
- min: minute
- µg: microgram
- mg: milligram
- MgSO₄: magnesium sulfate
- mL: microliter
- µm: micrometer
- µM: micromolar
- mm: millimeter
- mmol: millimol
- mL: milliliter
- mm: millimeter
- mol: mole
- mp: melting point
- MS: mass spectrometry
- m/z: mass to charge ratio
- MTBE: methyl tert-butyl ether
- N: normal
- NaHCO₃: sodium bicarbonate
- NaHMDS: sodium bis(trimethylsilyl)amide
- NaOH: sodium hydroxide
- Na₂SO₄: sodium sulfate
- NCS: n-chlorosuccinimide
- NH₄Cl: ammonium chloride
- NH₄OH: ammonium hydroxide
- NMR: nuclear magnetic resonance
- nM: nanomolar
- PCC: pyridinium chlorochromate
- Pd/C: palladium on carbon
- POCl₃: Phosphorous oxychloride
- P₂O₅: phosphorous pentoxide
- psi: pounds per square inch
- Rf: TLC retention factor
- rt: room temperature
- SPA: Scintillation Proximity Assay
- THF: tetrahydrofuran
- TFA: trifluoroacetic acid
- TMS: tetramethylsilane
- TLC: thin layer chromatography
- t_{R}: HPLC retention time

## Claims

1. A compound of the formula (I) 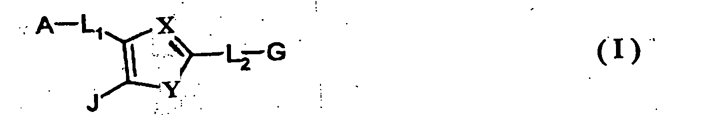 wherein
X represents N, O, C or S
Y represents S, NH, C or O
L¹ represents
a chemical bond;
carbonyl;
-(CH₂)ₐ- wherein
a is 1, 2, 3, 4 or 5;
-CH₂O-;
-OCH₂-;
-O- or -S-;
-N(R¹)- wherein
R¹ represents H, C₁₋₄ alkyl or NH;
-NHC(O)-; 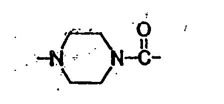 -CH₂NHC(O)-;
L² represents
a chemical bond;
-(CH₂)ₐ-;
-CH₂O-;
-N(R¹)-;
-NH(CH₂)ₐ-;
-N(CO)R¹-; or
-NHC(O)NH-;
J represents
H;
C₁₋₄ alkyl, wherein C₁₋₄ may be substituted by at least one halogen; or
halogen; and
A represents a (hetero)cyclic, aromatic or non-aromatic optionally substituted ring system;
G represents a (hetero)cyclic, aromatic or non-aromatic optionally substituted ring system or
L¹, A and J form together a cyclic or heterocyclic mono-, bi- or tricyclic ring system
or a pharmaceutically acceptable salt thereof.

2. Compound of formula (I), wherein A and G are independently from each other a mono-, bi- or polycyclic ring system.

3. Compound of formula (I) according to claims 1 or 2 wherein A and G represent independently from each other a mono- or bicyclic aryl or heteroaryl ring system.

4. Compound of formula (I) according to claim 1 or 2, wherein A and G are independently from each other an aromatic ring system selected from a group consisting of: benzol, pyridine, furane, pyrrole, thiophene, pyrazole, imidazole, thiazole, oxazole, pyridazine, pyrimidine, pyrazine, naphthaline, chinoline, isochinoline, indole, purine, benzofuran, phenanthrene.

5. Compound of formula (I) according to claim 1 or 2, wherein A and G are independently from each other C₃₋₈ cycloalkyl, C₃₋₈ heterocycloalkyl, C₅₋₆ (hetero)cycloalkenyl, C₆₋₁₂ (hetero)bicyloalkyl or C₆₋₁₂ (hetero)bicycloalkenyl.

6. Compound of formula (I) according to claims 4 or 5, wherein A and G are independently from each other substituted by one or more substituent(s) selected from the group consisting of:
C₁₋₆ alkyl;
C₁₋₄ haloalkyl;
OR¹;
C₃₋₆ cycloalkyl;
halogen;
phenyl optionally substituted by halogen;
NO₂;
CN;
-N(R³)₂; wherein
R³ represents H, C₁₋₄ alkyl, C₄₋₆ cycloalkyl, or phenyl
optionally substituted by halogen;
-(CH₂)ₐN(R¹)(R⁴); wherein
R⁴ represents -(CH₂)ₐOR¹ or -(CH₂)ₐN(R¹)₂; and
C₁₋₄ acyl; and
CO₂R¹; wherein R1 represents H, C₁₋₄ alkyl, C₁₋₄ haloalkyl, phenyl, benzyl optionally substituted by C₁₋₃ alkyl or C₁₋₃ haloalkyl

7. Compound of formula (I) according to any of the afore-mentioned claims, wherein A and G are - independently from each other - substituted at 0, 1 or 2 position(s).

8. Compound of formula (I) according to any of the afore-mentioned claims, wherein
X represents N
Y represents S, O or NH.

9. Compound of formula (I) according to any of the afore-mentioned claims, wherein
X represents N
Y represents S.

10. Compound of formula (I) according to any of the aforementioned claims, wherein L² represents -NH(CH₂)ₐ-, -N(R¹)-, -NHC(O)NH- or -N(CO)R¹-.

11. Compound of formula (I) according to any of the aforementioned claims, wherein L² represents -NH(CH₂)ₐ-.

12. Compound of formula (I) according to any of the aforementioned claims, wherein
A represents a mono- or bicyclic aryl or heteroaryl ring system, optionally substituted;
G represents a poly- or bicyclic or (hetero)aryl ring system or imidazole, pyrrole, thiophene, pyridazine, pyrazine, thiazole, or oxazole, optionally substituted;
L² represents -NH(CH₂)ₐ-, -NR¹-, -NHC(O)NH- or -NH(CO)R¹- ;
L¹ represents a chemical bond, carbonyl or -(CH₂)ₐ-.

13. Compound of formula (I) according to claim 12, wherein G is a poly- or bicyclic (hetero)aryl ring system under the provision that L¹ is a chemical bond.

14. Compound of formula (I) according to any of claims 1 - 11, wherein
• A represents a bicyclic aryl or heteroaryl ring system, optionally substituted,
with the provision that L¹ is carbonyl;
• A represents a bicyclic aryl or heteroaryl ring system, pyrrole, furan, thiophene, pyridine, pyridazine, pyrazine, pyrimidine, pyrazole, thiazole, or oxazole, optionally substituted, or
a non-aromatic C₃₋₁₂ mono- or bicyclic alkyl or heteroalkyl ring, optionally substituted,
with the provision that L¹ is -(CH₂)ₐ-; or
• A represents a bicyclic heteroaryl ring system, optionally substituted with the provision that A cannot be benzothiophene, or
a substituted bicyclic aryl ring system or
pyrrole, pyridazine, pyrazine, pyrimidine, thiazole, pyrazole, or imidazole, optionally substituted,
with the provision that L¹ is a chemical bond;
G represents a mono- or bicyclic aryl or heteroaryl ring system, optionally substituted; and
L² represents -NH(CH₂)ₐ- , -NR¹-, -NHC(O)NH-, or -NH(CO)R¹-.

15. A pharmaceutical composition comprising at least one compound of one of claims 1-14 and a pharmaceutically acceptable carrier.

16. Compound of any of claims 1 to 14 or composition according to claim 15 as kinase inhibitor, particularly as Src family kinase inhibitor.

17. Use of a compound of any of claims 1 to 14 or of a composition of claim 15 or 16 for the preparation of a medicament for the treatment of disease associated with an abnormal activity or level of a kinase, in particular cancer, immunosuppression, osteoporosis, allergic reactions.

18. Use according to claim 17 for the treatment of solid cancer, e.g. lung, kidney, breast, pancreas, skin, eye, bone, liver, prostate, ovarian or brain cancer or non-solid forms of cancer, e.g. tumors of the blood forming system.

19. Method of inhibiting a kinase, in particular a Src family kinase inhibitor, comprising contacting a kinase with a compound of any of claims 1 - 14.
